# EUROPEAN PATENT APPLICATION

(11) **EP 4 545 513 A1**
(43) Date of publication of application: **30.04.2025**
(21) Application number: 24203284.5
(22) Date of filing: 27.09.2024
(51) Int. Cl.: C07C 241/04, C07C 243/34

(54) **PREPARATION OF A CRYSTALLINE FORM OF AN ANTI-PARKINSON DRUG**

(30) Priority: 29.09.2023 IT 202300020187
(71) Applicant: Dipharma Francis S.r.l., 20021 Baranzate (MI) (IT)
(72) Inventor: ROSSI, Davide, 20021 BARANZATE (MI) (IT); RESTELLI, Alessandro, 20021 BARANZATE (MI) (IT); D'IMPERIO, Nicolas, 20021 BARANZATE (MI) (IT); VLADISKOVIC, Chiara, 20021 BARANZATE (MI) (IT); RAZZETTI, Gabriele, 20021 BARANZATE (MI) (IT)
(74) Representative: Bianchetti & Minoja with Trevisan & Cuonzo IPS SRL

(57) **Abstract**

The present invention relates to the preparation of a crystalline form of a DOPA decarboxylase inhibitor.

## Description

### TECHNICAL FIELD OF THE INVENTION

The present invention relates to the preparation of a crystalline form of a DOPA decarboxylase inhibitor.

### STATE OF THE ART

2-Amino-3-hydroxy-N'-(2,3,4-trihydroxybenzyl)propanehydrazide hydrochloride, also known as benserazide hydrochloride of formula **(I),** 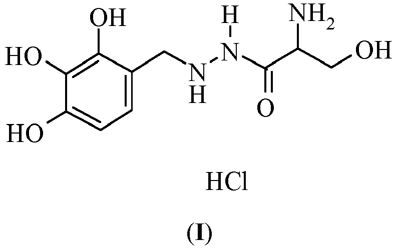 is used in the treatment of Parkinson's disease in combination with L-DOPA. Benserazide hydrochloride of formula **(I)** inhibits the conversion of L-DOPA to dopamine in extracerebral tissues, and in that way, it can reduce the occurrence of extracerebral side effects.

Benserazide hydrochloride of formula **(I)** can be prepared starting from DL-serine as described in US 3,178,476 (Scheme 1).

Reaction of 2,3,4-trihydroxybenzaldehyde with DL-serinoidrazide monohydrochloride in water, methanol, ethanol or in a mixture thereof provides the dihydrate form of (DL)-2-amino-3-hydroxy-N [(E)-(2,3,4-trihydroxyphenyl)methyleneamino]propanamide. If the reaction is carried out in an anhydrous solvent, such as absolute ethanol, the anhydrous form of the hydrazone is obtained. The subsequent hydrogenation with Pd/C in methanol furnishes benserazide hydrochloride of formula **(I):**

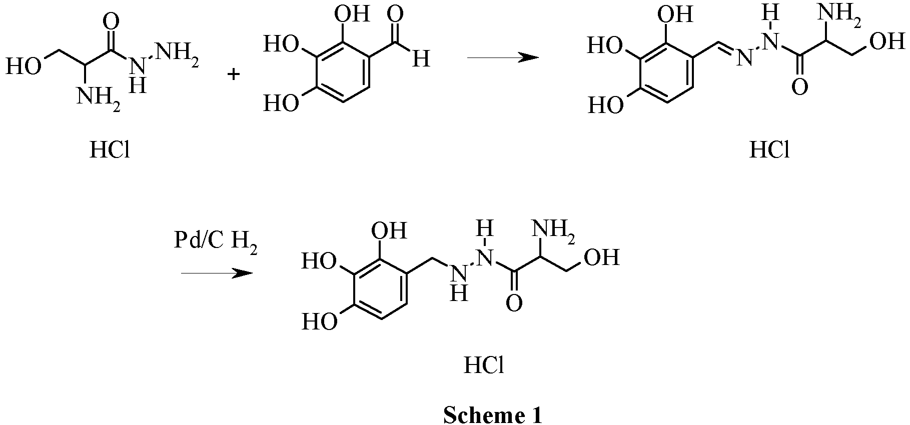

The commercial tablet formulation of benserazide hydrochloride of formula **(I)** contains the compound in crystalline form I. The XRPD of the crystalline form I is reported in WO 2015/197909.

WO 2015/197909 describes additional crystalline forms of benserazide hydrochloride of formula **(I),** e.g. crystalline form VI, obtained by recrystallization from an aqueous solution of ethanol (90%) and 1-propanol, or the DMF solvate, named crystalline form IX. Said crystalline form IX being a DMF solvate is not a suitable crystalline form for preparing a drug. According to the ICH guidelines on residual solvents present in active pharmaceutical ingredients, DMF is a class 2 solvent.

WO 2015/197909 also describes the preparation of crystalline form I by treating the DMF solvate with a mixture of aqueous ethanol (90%) and 1-propanol. The obtained product is washed with 1-propanol until DMF is no longer detected by HPLC analysis. However, HPLC analysis is not suitable for finding traces of DMF. For example, Gan *et al.* developed an HPLC method to determine DMF and reports a detection limit of 0.019% (Gan et al. Se Pu. 2011, Feb;29(2):184-6. doi: 10.3724/sp.j.1123.2011.00184.). In addition, in view of a recently published EU regulation, EU 2021/2030, also the legislators attempt to minimize the use of DMF and thus there is a need for identifying methods, which do not use DMF. Consequently, due to the toxicity of DMF, alternative methods are needed for the preparation of benserazide hydrochloride in crystalline form I, which are efficient, cost-effective, and suitable for an industrial scale production and purification.

The authors of the present invention have found a new and safe method for preparing benserazide hydrochloride of formula **(I)** in crystalline form I, which thanks to the high yields and a lower presence of impurities, in particular non-toxic solvents, is particularly suitable for an industrial production. This new process provides a pure product, which is suitable to meet the regulatory requirements mandatory for active pharmaceutical ingredients (APIs).

The herein disclosed process for preparing benserazide hydrochloride of formula **(I)** in crystalline form I does not make use of methanol and/or DMF as crystallization solvent and allows to eliminate any methanol from the finished product, if used as a solvent during the synthesis of benserazide hydrochloride of formula **(I).**

### SUMMARY OF THE INVENTION

In a first aspect, the present invention concerns a process for preparing benserazide hydrochloride of formula **(I)** in crystalline form I, 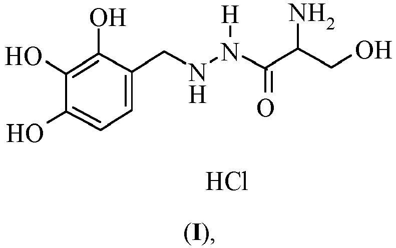 wherein the characteristic XRPD peaks, measured using CuKα radiation, are found at 7.6; 11.0; 12.2; 18.5; 18.9; 21.8; 23.1; 24.6 and 25.4° ± 0.2° in 2θ, starting from benserazide hydrochloride of formula **(I)** in crystalline form VI, whose characteristic peaks, obtained using CuKα radiation, are found at 18.6; 22.7; 23.0; 25.5; 27.2; 27.9; 33.1 and 34.6° ± 0.2° in 2θ.

This process is particularly suitable for obtaining benserazide hydrochloride of formula **(I)** in crystalline form I that does not contain methanol or wherein the methanol content is lower than 500 ppm (parts per million), which is an acceptable amount according to ICH guidelines on residual solvents.

### BRIEF DESCRIPTION OF FIGURES AND ANALYTICAL METHODS

The crystalline forms I, VI and α of benserazide hydrochloride of formula (I) were characterized by X-ray powder diffraction (XRPD).

The water content was determined by titration using the Karl Fischer technique.

X-ray diffraction spectra (XRPD) were run on a Bruker D8 Advance diffractometer having a goniometer radius of 280 mm and working in the following conditions: CuKα radiation filtered by Nickel filter (λ = 1.54 Å), Bragg-Brentano geometry, scanning with angular range 3-40° in 20 with angular pitch of 0.02°. The diffractogram obtained was evaluated using Diffrac. Eva version 4.3.0.1 (Bruker AXS).
**Figure 1****:** XRPD spectrum of benserazide hydrochloride of formula (I) in crystalline form I.
**Figure 2****:** XRPD spectrum of benserazide hydrochloride of formula (I) in crystalline form VI.
**Figure 3****:** XRPD spectrum of benserazide hydrochloride of formula (I) in crystalline form α.

### DETAILED DESCRIPTION OF THE INVENTION

In a first aspect, the present invention concerns a process for preparing benserazide hydrochloride of formula **(I)** in crystalline form I, 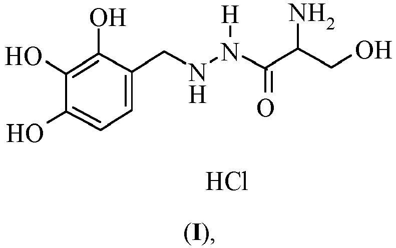 wherein the characteristic XRPD peaks, measured using CuKα radiation, are found at 7.6; 11.0; 12.2; 18.5; 18.9; 21.8; 23.1; 24.6 and 25.4° ± 0.2° in 2θ, starting from benserazide hydrochloride of formula **(I)** in crystalline form VI, whose characteristic peaks, obtained using CuKα radiation, are found at 18.6; 22.7; 23.0; 25.5; 27.2; 27.9; 33.1 and 34.6° ± 0.2° in 2θ.

This process is particularly suitable for obtaining benserazide hydrochloride of formula **(I)** in crystalline form I that does not contain methanol or wherein the methanol content is lower than 500 ppm (parts per million), which is an acceptable amount according to ICH guidelines on residual solvents.

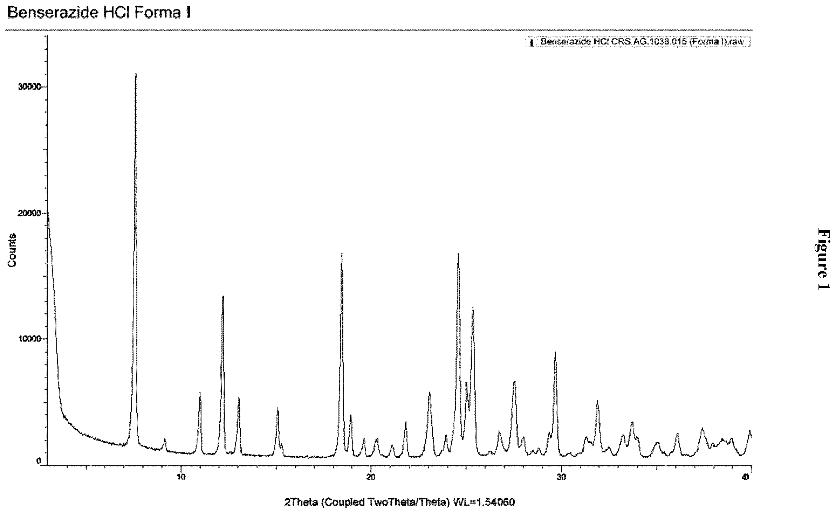

Benserazide hydrochloride of formula **(I)** in crystalline form I is characterized by X-ray diffraction, wherein the characteristic XRPD peaks, measured using CuKα radiation, are as substantially shown in Figure 1 and which is characterized by the following characteristic peaks:

**Table 1**

| 2θ ± 0.2 [°] | d-spacing [Å] | Relative intensity [I/I] |
|---|---|---|
| 7.62 | 11.59 | 1.00 |
| 11.02 | 8.02 | 0.16 |
| 12.20 | 7.25 | 0.43 |
| 13.05 | 6.78 | 0.16 |
| 15.09 | 5.86 | 0.13 |
| 18.46 | 4.80 | 0.55 |
| 18.93 | 4.68 | 0.11 |
| 21.83 | 4.07 | 0.10 |
| 23.06 | 3.85 | 0.17 |
| 24.58 | 3.62 | 0.54 |
| 25.35 | 3.51 | 0.40 |

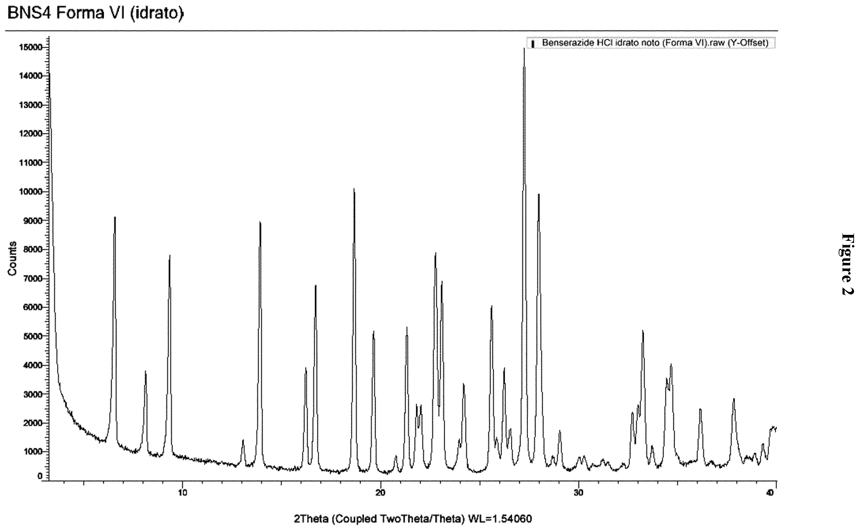

Benserazide hydrochloride of formula **(I)** in crystalline form VI is characterized by an XRPD as substantially shown in Figure 2.

According to one aspect, benserazide hydrochloride of formula **(I)** in crystalline form I, 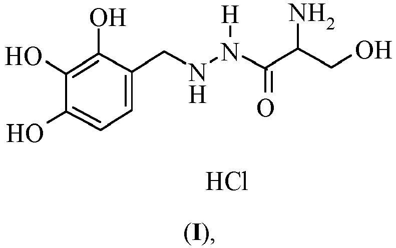 wherein the characteristic XRPD peaks, measured using CuKα radiation, are found at 7.6; 11.0; 12.2; 18.5; 18.9; 21.8; 23.1; 24.6 and 25.4° ± 0.2° in 2θ, is prepared by a process comprising:
- forming a dispersion of benserazide hydrochloride of formula **(I)** in crystalline form VI, wherein the characteristic XRPD peaks, measured using CuKα radiation, are found at 18.6; 22.7; 23.0; 25.5; 27.2; 27.9; 33.1 and 34.6° ± 0.2° in 2θ, in ethanol or in a mixture of ethanol and isopropanol,
- maintaining the dispersion in ethanol or in an ethanol and isopropanol mixture for 2 hours or more than 2 hours, and
- recovering benserazide hydrochloride of formula **(I)** in crystalline form I.

According to the present invention, the term "comprising" means that additional reaction steps may be present, but which do not substantially modify the product obtained from the process. The term "comprising" also includes the terms "consisting of" and "essentially consisting of".

According to the present invention, the term "forming a dispersion of benserazide hydrochloride of formula **(I)** in crystalline form VI" as used herein means a partial dissolution of benserazide hydrochloride of formula **(I)** in crystalline form VI, thus the dispersion of benserazide hydrochloride of formula **(I)** in crystalline form VI does not lead to a complete dissolution of the benserazide hydrochloride of formula **(I)** in crystalline form VI.

According to the Japanese Pharmacopoeia, benserazide hydrochloride of formula **(I)** results to be soluble in water and in formic acid, soluble in methanol, but only very slightly soluble in ethanol.

The residual methanol content of benserazide hydrochloride of formula **(I)** in crystalline form VI used for preparing crystalline form I measured by GC-HS can be about 50,000 ppm or less, for example 20,000 ppm or less, 10,000 ppm or less, 5,000 ppm or less, or 1,000 ppm or less.

In one aspect, the residual methanol content of benserazide hydrochloride of formula **(I)** in crystalline form VI used for preparing crystalline form I measured by GC-HS may be between about 1 and about 20,000 ppm, for instance between about 10 and about 10,000 ppm or between 50 and 5,000 ppm.

The ratio between benserazide hydrochloride of formula **(I)** in crystalline VI and ethanol or the ethanol and isopropanol mixture may be between 0.1% and 70% (w:w, weight:weight), preferably between 1% and 50%, e.g. 5%, 10%, 15%, 20%, 25%, 30%, 35% or 40% (w:w).

The dispersion of benserazide hydrochloride of formula **(I)** in crystalline form VI in ethanol or in a mixture of ethanol and isopropanol may be advantageously carried out at a temperature between about 15°C and about 30°C, preferably between about 15°C and about 25°C, for example at about 20°C or at about 25°C.

The dispersion of benserazide hydrochloride of formula **(I)** in crystalline form VI in ethanol or in an ethanol and isopropanol mixture may be maintained for two hours or for more than two hours, e.g. for 3 hours, 4 hours, 5 hours, 6 hours, 7 hours, 8 hours, 9 hours, 10 hours, 11 hours, 12 hours, 14 hours, 16 hours, 18 hours, 24 hours, 36 hours, 48 hours, 72 hours, 96 hours, or for 144 hours.

In one aspect, the dispersion of benserazide hydrochloride of formula **(I)** in crystalline form VI in ethanol or in an ethanol and isopropanol mixture may be stirred for 8 hours, 16 hours or for 24 hours.

In one aspect, the dispersion of benserazide hydrochloride of formula **(I)** in crystalline form VI may be carried out in ethanol.

In one aspect, the formation of the dispersion of benserazide hydrochloride of formula **(I)** in crystalline form VI may be carried out in a mixture of ethanol and isopropanol.

In one aspect, the formation of the dispersion of benserazide hydrochloride of formula **(I)** in crystalline form VI may be carried out in a mixture consisting of ethanol and isopropanol.

The ratio between ethanol and isopropanol may be between about 1:1000 and 1000:1 (v:v, volume:volume).

The ratio between ethanol and isopropanol may be between about 1:1000 and 1000:1 (v:v), for example, between about 1:100 and 100:1 (v:v), or 10:1 and 1:10 (v:v), for example 1:1, 2:1, 3:1, 4:1, 5:1, 6:1, 7:1, 8:1, 9:1 or 1:2, 1:3, 1:4, 1:5, 1:6, 1:7, 1:8 or 1:9 (v:v).

The ratio between ethanol and isopropanol may be, for example, between about 1:100 and 100:1 (v:v).

The ratio between ethanol and isopropanol may be, for example, between about 10:1 and 1:10 (v:v).

The ratio between ethanol and isopropanol may be, for example, 1:1, 2:1, 3:1, 4:1, 5:1, 6:1, 7:1, 8:1, 9:1, 10:1 or 1:2, 1:3, 1:4, 1:5, 1:6, 1:7, 1:8, 1:9, or 1:10 (v:v).

In a preferred aspect, the ratio between ethanol and isopropanol may be, for example, between about 1:100 and 100:1 (v:v), more preferably between about 10:1 and 1:10 (v:v), for example 1:1, 2:1, 3:1, 4:1, 5:1, 6:1, 7:1, 8:1, 9:1 or 1:2, 1:3, 1:4, 1:5, 1:6, 1:7, 1:8, or 1:9 (v:v).

The suspension of benserazide hydrochloride of formula **(I)** in ethanol or in the ethanol and isopropanol mixture may be optionally cooled down, for example to between about 5°C to about 25°C, preferably to between about 7°C and about 25°C, for example to about 10°C, to about 15°C, to about 20°C or to about 25°C.

If desired, a previously obtained seed of benserazide hydrochloride of formula **(I)** in crystalline form I may be added to the suspension of benserazide hydrochloride of formula **(I)** in ethanol or in the ethanol and isopropanol mixture.

The recovery of the solid, consisting of benserazide hydrochloride of formula **(I)** in crystalline form I may be carried out according to known techniques, for example by filtration or by centrifugation, preferably by filtration.

Benserazide hydrochloride of formula **(I)** in crystalline form I may be dried according to known methods.

For example, the drying may be carried out at reduced pressure or under air or nitrogen flow.

In a preferred aspect, the drying is carried out at a pressure of less than 0.5 bar.

The drying temperature may be between room temperature and 70°C, preferably between 25 and 60°C, for example at 40°C, at 45°C or at 50°C.

The size of the crystals of benserazide hydrochloride of formula **(I)** in crystalline form I obtained according to the described process is characterized by a value of D50 between 5 µm and 250 µm. If desired, the size may be reduced by a micronization step or by fine milling/grinding.

Benserazide hydrochloride of formula **(I)** in crystalline form I as obtainable according to the invention, has a high purity, typically ≥ 99% (area) measured at 210 nm in HPLC, and the impurities are present in an amount lower than or equal to 0.1%, preferably less than or equal to 0.05% measured at 210 nm in HPLC.

The obtained benserazide hydrochloride of formula **(I)** in crystalline form I has a water content between about 0 and about 1% (w:w), preferably between about 0.05 and 0.9% (w:w), so that it can be defined to be substantially anhydrous.

In addition, thanks to the fact that methanol or DMF is not used in the crystallization process of benserazide hydrochloride, the so obtained benserazide hydrochloride of formula **(I)** in crystalline form I does not contain methanol or the methanol content is lower than 500 ppm, preferably lower than 300 ppm, more preferably lower than 100 ppm, for example 0 ppm, 4.5 ppm, 10 ppm or 50 ppm.

A further aspect of the present invention concerns a crystallization process for preparing benserazide hydrochloride of formula **(I)** without using methanol or DMF as solvent.

A further aspect of the present invention concerns a crystallization process for preparing benserazide hydrochloride of formula **(I)** without using methanol and DMF as solvent.

Benserazide hydrochloride of formula **(I)** in crystalline form VI is known from WO 2015/197909, which describes its preparation starting from the DMF solvate, named also crystalline form IX.

Since DMF according to ICH guidelines is part of the group of solvents to be avoided (DMF is a member of class 2 solvents), the inventors surprisingly found a method to prepare benserazide hydrochloride of formula **(I)** in crystalline form VI and form I employing only water, ethanol and isopropanol.

In one aspect, benserazide hydrochloride of formula **(I)** in crystalline form VI may be prepared by a process comprising:
- forming a solution of benserazide hydrochloride of formula **(I)** in water,
- adding isopropanol to the obtained aqueous solution,
- recovering benserazide hydrochloride of formula **(I)** in crystalline form VI.

Benserazide hydrochloride of formula **(I)** used as starting material for preparing benserazide hydrochloride of formula **(I)** in crystalline form VI may be in any of its known forms. For instance, it may be amorphous or crystalline benserazide hydrochloride. For example, benserazide hydrochloride of formula **(I)** may be in crystalline form I.

The dissolution of benserazide hydrochloride of formula **(I)** in water may be advantageously carried out at a temperature between about 15°C to 30°C, preferably between about 15°C and about 25°C, for example at about 20°C or at about 25°C.

The dissolution of benserazide hydrochloride of formula **(I)** in water may be advantageously carried out within 15 minutes or in more than 15 minutes, for example within 30 minutes, within 45 minutes, within 1 hour, within 2 hours, within 3 hours, within 4 hours, or within 5 hours.

In one aspect, the dissolution time of benserazide hydrochloride of formula **(I)** in water before adding isopropanol is 15 minutes or more than 15 minutes, for example 30 minutes, 45 minutes, 1 hour, or 2 hours.

In one aspect, the dissolution time of benserazide hydrochloride of formula **(I)** in water before adding isopropanol is less than two hours.

In a preferred aspect, the dissolution time of benserazide hydrochloride of formula **(I)** in water before adding isopropanol is less than one hour.

In one aspect, the ratio between water and isopropanol may be, for example, between about 1000:1 and 1:2 (v:v), for example 100:1, 10:1, or 1:1 (v:v).

The suspension of benserazide hydrochloride of formula **(I)** in water and isopropanol may be optionally cooled down, e.g. to between about 5°C to 25°C, preferably between about 7°C and about 25°C, for example at about 10°C, about 15°C, about 20°C or at about 25°C.

The suspension of benserazide hydrochloride of formula **(I)** in water and isopropanol may be stirred for one hour or for more than one hour, for example for 2 hours, 3 hours, 4 hours, 5 hours, 6 hours, 7 hours, 8 hours, 9 hours, 10 hours, 11 hours, 12 hours, 18 hours, 24 hours, 48 hours, 72 hours or for 96 hours.

Before separating the solid, the solution may be cooled down to increase the yields. The cooling of the solution may be carried out at a temperature lower than or equal to about 20°C, preferably between about 0°C and about 20°C, more preferably between about 5°C and about 10°C. The cooling may be done slowly, for example at a rate of between about 0.1°C and 0.4°C per minute.

If desired, a previously obtained seed of the desired crystalline form may be added.

The separation of the solid, consisting of benserazide hydrochloride of formula **(I)** in crystalline form VI may be carried out by known techniques, for example by filtration or by centrifugation, preferably by filtration.

Benserazide hydrochloride of formula **(I)** in crystalline form VI may be dried according to known methods.

For example, drying may be carried out at reduced pressure or under air or nitrogen flow.

Preferably, drying is carried out at a pressure of less than 0.5 bar.

The drying temperature may be between room temperature and 70°C, preferably between 25°C and 55°C, for example at 45°C.

In another aspect, the present disclosure relates to a crystalline form of benserazide hydrochloride of formula **(I),** called crystalline form α,

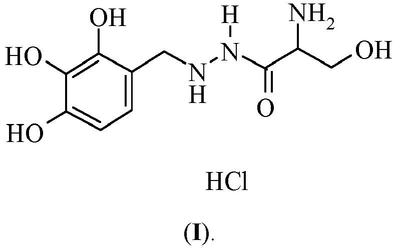

According to a further aspect, the characteristic XRPD peaks of benserazide hydrochloride of formula **(I)** in crystalline form α, obtained using CuKα radiation, are found at 8.6; 9.2; 13.9; 17.2 and 18.4° ± 0.2° in 2Θ.

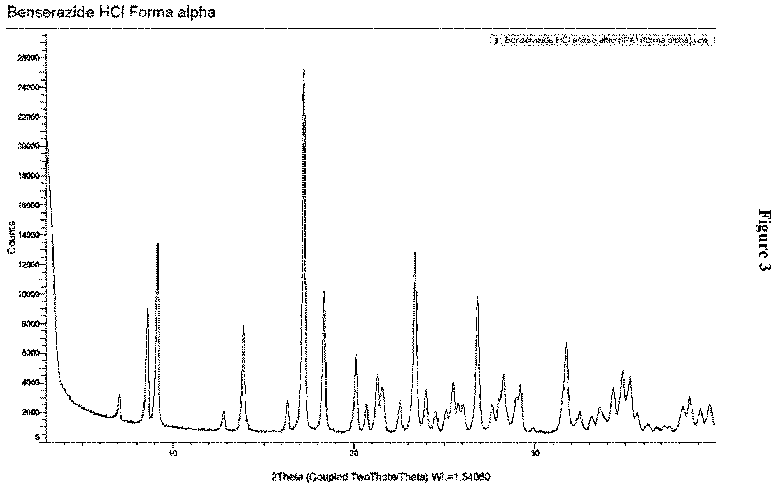

In a preferred aspect, benserazide hydrochloride of formula **(I)** in crystalline form α is characterized by XRPD as substantially shown in Figure 3 and which includes further peaks, obtained using CuKα radiation, substantially at 7.1; 12.8; 16.3; 20.1; 20.7; 21.3; 21.6; 22.6; 23.4; 24.0; 24.5; 25.1; 25.5; 25.8; 26.1; 26.8; 27.7; 28.0; 28.3; 29.0; 29.2; 29.9; 31.7; 32.5 and 33.1° ± 0.2° in 2θ.

According to a further aspect, benserazide hydrochloride of formula **(I)** in crystalline form α is characterized by X-ray diffraction, wherein the characteristic XRPD peaks, measured using CuKα radiation, are as substantially shown in Figure 3 and which is characterized by the following characteristic peaks:

**Table 2**

| **2θ ± 0.2 [°]** | d-spacing [Å] | Relative intensity [I/I] |
|---|---|---|
| 7.1 | 12.48 | 0.07 |
| 8.6 | 10.25 | 0.32 |
| 9.2 | 9.64 | 0.50 |
| 12.8 | 6.90 | 0.05 |
| 13.9 | 6.36 | 0.29 |
| 16.3 | 5.42 | 0.08 |
| 17.2 | 5.14 | 1.00 |
| 18.4 | 4.83 | 0.39 |
| 20.1 | 4.41 | 0.21 |
| 20.7 | 4.29 | 0.07 |
| 21.3 | 4.17 | 0.16 |
| 21.6 | 4.12 | 0.12 |
| 22.6 | 3.94 | 0.08 |
| 23.4 | 3.80 | 0.50 |
| 24.0 | 3.71 | 0.11 |
| 24.5 | 3.63 | 0.06 |
| 25.1 | 3.54 | 0.05 |
| 25.5 | 3.49 | 0.13 |
| 25.8 | 3.45 | 0.07 |
| 26.1 | 3.42 | 0.07 |
| 26.8 | 3.32 | 0.37 |
| 27.7 | 3.22 | 0.07 |
| 28.0 | 3.18 | 0.08 |
| 28.3 | 3.15 | 0.15 |
| 29.0 | 3.08 | 0.09 |
| 29.2 | 3.06 | 0.13 |
| 29.9 | 2.98 | 0.01 |
| 31.7 | 2.82 | 0.25 |
| 32.5 | 2.76 | 0.05 |

According to a further aspect, benserazide hydrochloride of formula (I) in crystalline form α, 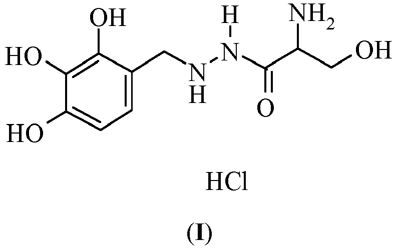 may be prepared by a process comprising:
- forming a solution of benserazide hydrochloride of formula **(I)** in water,
- adding isopropanol to the obtained aqueous solution, and wherein the ratio of water to isopropanol may be, for example, between approximately 1:3 and 1:100 (v:v), and
- recovering benserazide hydrochloride of formula **(I)** in crystalline form α.

Benserazide hydrochloride of formula **(I)** used as starting material may be in one of its known forms, it may be amorphous or crystalline benserazide hydrochloride. For example, benserazide hydrochloride of formula **(I)** may be in crystalline form I.

The ratio between benserazide hydrochloride of formula **(I)** and water may be between 0.1% and 70% (w:w), preferably between 10% and 50% (w:w), for example 15%, 20%, 25%, 30%, 35% or 40% (w:w).

The dissolution of benserazide hydrochloride of formula **(I)** in water may be advantageously carried out for example between about 15°C and about 30°C, preferably between about 15°C and about 25°C, for example at about 20°C or at about 25°C.

The dissolution of benserazide hydrochloride of formula **(I)** in water may be advantageously carried out within 15 minutes or in more than 15 minutes, for example within 30 minutes, within 45 minutes, within 1 hour, within 2 hours, within 3 hours, within 4 hours, or within 5 hours.

In one aspect, the dissolution time of benserazide hydrochloride of formula **(I)** in water before adding isopropanol is 15 minutes or more than 15 minutes, such as 30 minutes, 45 minutes, 1 hour, or 2 hours.

In one aspect, the dissolution time of benserazide hydrochloride of formula **(I)** in water before adding isopropanol is less than two hours.

In a preferred aspect, the dissolution time of benserazide hydrochloride of formula **(I)** in water before adding isopropanol is less than one hour.

The ratio between benserazide hydrochloride of formula **(I)** and isopropanol may be between 0.1 g and 50 g of benserazide hydrochloride of formula **(I)** per 100 mL of isopropanol, preferably between 2 g and 30 g, for example 5 g, 10 g, 15 g, or 20 g of benserazide hydrochloride of formula **(I)** per 100 mL of isopropanol.

The ratio between water and isopropanol may be, for example, between about 1:3 and 1:100 (v:v), preferably between about 1:3 and 1:50 (v:v), for example 1:4 (v:v) or 1:5 (v:v).

The solution comprising benserazide hydrochloride of formula **(I)** in water and isopropanol may be optionally cooled down, e.g. to between about 5°C to 25°C, preferably to between about 7°C and about 25°C, e.g. at about 10°C, about 15°C, about 20°C or at about 25°C.

The suspension of benserazide hydrochloride of formula **(I)** in water and isopropanol may be maintained for one hour or more than one hour, e.g. for 2 hours, 3 hours, 4 hours, 5 hours, 6 hours, 7 hours, 8 hours, 9 hours, 10 hours, 11 hours, 12 hours, 18 hours, 24 hours, 48 hours, 72 hours or for 96 hours.

Before separating, the dispersion may be cooled down to increase the yield. The dispersion may be cooled down to a temperature lower than or equal to about 20°C, preferably between about 0°C and about 20°C, more preferably between about 5°C and about 10°C. The cooling may be done slowly, for example at a rate of between about 0.1°C and 0.4°C per minute.

If desired, a previously obtained seed may be added.

The separation of the solid, consisting of benserazide hydrochloride of formula **(I)** in crystalline form α may be carried out by known techniques, for example by filtration or by centrifugation, preferably by filtration.

Benserazide hydrochloride of formula **(I)** in crystalline form α may be dried according to known methods.

For example, the drying step may be carried out at reduced pressure or under air or nitrogen flow. Preferably, drying is carried out at a pressure of less than 0.5 bar.

The drying temperature may be between room temperature and 70°C, preferably between 25 and 55°C, for example at 45°C.

Benserazide hydrochloride of formula **(I)** in crystalline form α prepared according to the present disclosure, has a high purity, typically ≥ 99% measured at 210 nm in HPLC.

The obtained benserazide hydrochloride of formula **(I)** in crystalline form α has a water content between about 0 and about 1% (w:w), preferably between about 0.05 and 0.9% (w:w), so that it may be defined to be substantially anhydrous.

A further aspect of the present disclosure relates to a pharmaceutical composition comprising benserazide hydrochloride of formula **(I)** in crystalline form α, as an active ingredient, and one or more pharmaceutically acceptable excipients and/or vehicles.

The following examples further illustrate the invention.

### Example 1. Preparation of Benserazide Hydrochloride of Formula (I) in Crystalline Form I

20 g (61 mmol) of 2-amino-3-hydroxy-*N*-[(E)-(2,3,4-trihydroxyphenyl)methyleneamino]propanamide, which can be prepared by treating 2,3,4-trihydroxybenzaldehyde with DL-serinoidrazide monohydrochloride as described in US 3,178,476, and 1.3 g of Pd/C in 460 mL of methanol are placed at 25°C in a 500 mL four-neck flask equipped with a magnetic stirrer. Subsequently, the mixture is vigorously stirred for 6 hours in a hydrogen atmosphere and at ambient conditions. The suspension is then cooled down to 5°C and filtered on celite. The panel is washed with 20 mL of methanol and the combined solution is concentrated by distillation at reduced pressure until a light orange oily residue is obtained.

100 mL of ethanol is added to the oily residue at 25°C and the light orange mixture is stirred at an internal temperature of 25°C. After about 30-60 minutes an off-white solid is formed. The mixture protected from light is stirred for 6 hours and then the solid is filtered off. The panel is washed 2 times with 20 mL of ethanol and the recovered white/off-white solid is dried in an oven at 45°C for 8 hours furnishing 13.4 g (yield 75%) of benserazide hydrochloride of formula **(I)** in crystalline form I.

The residual methanol content measured by GC-HS is 2500 ppm.

### Example 2. Preparation of Benserazide Hydrochloride of Formula (I) in Crystalline Form VI

20 g (61 mmol) of 2-amino-3-hydroxy-*N*-[(E)-(2,3,4-trihydroxyphenyl)methyleneamino]propanamide, which can be prepared by treating 2,3,4-trihydroxybenzaldehyde with DL-serinoidrazide monohydrochloride as described in US 3,178,476, and 1.3 g of Pd/C in 460 mL of methanol are loaded at 25°C in a 1000 mL four-neck flask equipped with a magnetic stirrer. Subsequently, the mixture is vigorously stirred for 6 hours in a hydrogen atmosphere and at ambient conditions. The suspension is then cooled down to 5°C and filtered on celite. The panel is washed with 20 mL of methanol and the combined solution is concentrated by distillation at reduced pressure until a light orange oily residue is obtained.

40 mL of water is added at 25°C to the residue and then 40 mL of isopropanol are added to the mixture within 30 minutes. The clear light-yellow solution is stirred at 25°C for 16 hours. If desired, a seed of 0.02 g of benserazide hydrochloride of formula **(I)** in crystalline form VI may be added to the solution. The suspension is cooled down to 3°C at a rate of 10°C per hour and kept at 3°C for two hours. The solid is then filtered off, the panel is washed with 20 mL of a isopropanol/water mixture (5:1, v:v) and with 20 mL of isopropanol, and dried in the oven at 45°C for 16 hours providing benserazide hydrochloride of formula **(I)** in crystalline form VI having a white/off-white color (yield 70%). The product is a monohydrate with a water content measured by Karl-Fischer analysis of 5-6% and with a purity of 99.40% (HPLC detector at 210 nm).

The residual methanol content measured by GC-HS is 900 ppm.

### Example 3. Preparation of Benserazide Hydrochloride of Formula (I) in Crystalline Form I

20 g (0.064 moles) of benserazide hydrochloride of formula **(I)** in crystalline form VI, obtained as described in Example 2, and 200 ml of an ethanol and isopropanol mixture (1:1, v:v) are loaded at 25°C in a 500 mL 4-neck reactor equipped with a mechanical stirrer, a reflux condenser, a thermometer and under nitrogen atmosphere. The suspension is stirred for 16 hours at 25°C and then filtered off. The panel is washed 2 times with 20 mL of a isopropanol/ethanol mixture (1:1, v:v) and the obtained white/off-white solid is dried under vacuum at 50°C for 8 hours providing 17.9-18.6 g (yield of 95-99%) of benserazide hydrochloride of formula **(I)** in crystalline form I with a water content measured by Karl-Fischer analysis of <1% and a purity of 99.51% (HPLC detector at 210 nm).

The residual methanol content measured by GC-HS is 0 to 5 ppm.

### Example 4. Preparation of Benserazide Hydrochloride of Formula (I) in Crystalline Form α

30 g (0.10 moles) of benserazide hydrochloride of formula **(I)** in crystalline form I, obtained as described in Example 1, and 60 mL of water are placed at 25°C in a 500 mL 4-neck reactor equipped with a mechanical stirrer, reflux condenser, thermometer and under nitrogen atmosphere. 300 mL of isopropanol is added to the light-yellow solution at 20-25°C and the mixture is stirred at 20-25°C until a precipitate is formed. After further 4 hours of stirring at 20-25°C, the resulting precipitate is filtered off. The panel is washed 2 times with 30 mL of isopropanol and the recovered white/off-white solid is dried in vacuum at 45°C for 16 hours furnishing 24.9 g (yield of 83%) of benserazide hydrochloride of formula **(I)** in crystalline form α with a water content measured by Karl-Fischer analysis of 0.68% and a purity of 99.22% (HPLC detector at 210 nm).

### Example 5. Preparation of Benserazide Hydrochloride of Formula (I) in Crystalline Form α

30 g (0.10 moles) of benserazide hydrochloride of formula **(I)** in crystalline form I, obtained as described in Example 1, and 75 mL of water are placed at 25°C in a 100 mL 4-neck reactor equipped with mechanical stirrer, reflux condenser, thermometer and under nitrogen atmosphere. 300 mL of isopropanol is added to the light-yellow solution at 20-25°C and the mixture is stirred at 20-25°C until a precipitate is formed. After further 4 hours of stirring at 20-25°C, the resulting product is filtered off. The panel is washed 2 times with 30 mL of isopropanol and the recovered white/off-white solid is dried in vacuum at 45°C for 16 hours furnishing 24 g (yield of 80%) of benserazide hydrochloride of formula **(I)** in crystalline form α with a water content measured by Karl-Fischer analysis of 0.89% and a purity of 99.24% (HPLC detector at 210 nm).

The residual methanol content measured by GC-HS is 4.5 ppm.

### Example 6. Benserazide Hydrochloride of Formula (I) in Crystalline Form α

Benserazide hydrochloride of formula **(I)** in crystalline form α, prepared as described in Example 4 or 5, suspended in an ethanol and isopropanol mixture (1:1, v:v) according to the conditions described in Example 3 or in isopropanol does not convert into another polymorph. Benserazide hydrochloride of formula **(I)** in crystalline form α results to be stable also in a water/ethanol/isopropanol mixture (0.02:1:1, v:v:v) or in a water /isopropanol mixture (0.01:1, v:v).

Thus, contrary to benserazide hydrochloride of formula **(I)** in crystalline form VI, benserazide hydrochloride of formula **(I)** in crystalline form I cannot be prepared by suspending crystalline form α in ethanol/isopropanol or in isopropanol, both in presence or in absence of water.

## Claims

1. A process for preparing benserazide hydrochloride of formula **(I)** in crystalline form I, 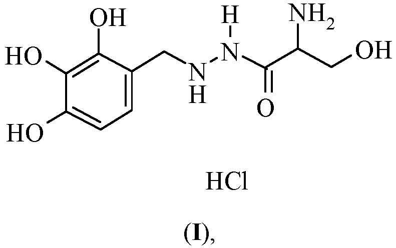 wherein the characteristic XRPD peaks, measured using CuKα radiation, are found at 7.6; 11.0; 12.2; 18.5; 18.9; 21.8; 23.1; 24.6 and 25.4° ± 0.2° in 2θ,
comprising:
• forming a dispersion of benserazide hydrochloride of formula **(I)** in crystalline form VI, wherein the characteristic XRPD peaks, measured using CuKα radiation, are found at 18.6; 22.7; 23.0; 25.5; 27.2; 27.9; 33.1 and 34.6° ± 0.2° in 2θ, in ethanol or in a mixture of ethanol and isopropanol,
• maintaining the dispersion in ethanol or in the mixture of ethanol and isopropanol for 2 hours or more than 2 hours, and
• recovering benserazide hydrochloride of formula **(I)** in crystalline form I.

2. The process according to claim 1, wherein the dispersion of benserazide hydrochloride of formula **(I)** in ethanol or in the mixture of ethanol and isopropanol is carried out at between 15°C to 30°C.

3. The process according to claims 1 and 2, wherein the dispersion of benserazide hydrochloride of formula **(I)** in crystalline form VI is carried out in a mixture consisting of ethanol and isopropanol.

4. The process according to claim 3, wherein the ratio of ethanol to isopropanol is between 1:1000 and 1000:1 (v:v).

5. The process according to claims 3 or 4, wherein the ratio of ethanol to isopropanol is between 1:10 and 10:1.

6. The process according to claims 1 to 5, without using methanol.

7. The process according to claims 1 to 6, further comprising:
• forming a solution of benserazide hydrochloride of formula (I) in water,
• adding isopropanol to the obtained aqueous solution,
• recovering benserazide hydrochloride of formula **(I)** in crystalline form VI.

8. The process according to claim 7, wherein the ratio between water and isopropanol is between 1000:1 to 1:2 (v:v).

9. Benserazide hydrochloride of formula **(I)** in crystalline form I, as defined in claim 1, wherein the content of methanol is less than 500 ppm compared to the content of benserazide hydrochloride.
